Europäisches Patentamt

European Patent Office

Office européen des brevets

(19)

(11) Publication number: **0 258 928 B1**

# EUROPEAN PATENT SPECIFICATION

(45) Date of publication of patent specification: **13.11.91**   (51) Int. Cl.⁵: **A61M 16/10**

(21) Application number: **87201563.1**

(22) Date of filing: **18.08.87**

(54) **Device for supplying air or medical gases in a conditioned, particularly a moistened and/or heated state to a patient.**

(30) Priority: **26.08.86 BE 217082**

(43) Date of publication of application:
**09.03.88 Bulletin 88/10**

(45) Publication of the grant of the patent:
**13.11.91 Bulletin 91/46**

(84) Designated Contracting States:
**CH DE ES FR GB IT LI NL SE**

(56) References cited:
**DE-A- 2 002 027**
**FR-A- 2 250 542**
**FR-A- 2 377 808**
**GB-A- 188 504**

(73) Proprietor: **"PONNET, GILMAN EN ANTHONY"**
**Vogelkerslaan 20**
**B-2080 Kapellen(BE)**

(72) Inventor: **Anthony, Jean-Michel**
**Ringlaan 78**
**B-2610 Wilrijk(BE)**

(74) Representative: **Pieraerts, Jacques et al**
**Bureau Gevers S.A. rue de Livourne 7 bte 1**
**B-1050 Bruxelles(BE)**

## Description

This invention relates to a device for supplying air or medical gases in a conditioned, particularly a moistened and/or heated state to a patient, comprising a tube, wherethrough the moistened air flows, and a cylinder-shaped element to which the tube (6) is connectable, self-regulating heating resistors being provided which heat the air flowing through said tube, while said tube is at least partly filled with a material which adsorbs or absorbs the water supplied by a water-supply line from a container, and has a good air-permeability, as disclosed in the FR-A-2 250 542

Such devices are known and are generally comprised of a water tank which insures a water supply to a generally cylinder-shaped chamber wherewith a supply line for an air/oxygen mixture and a discharge line for the heated and moistened mixture are connected. Depending on the local circumstances and requirements, the length of the discharge line, that is the line to the patient, may not always be determined beforehand.

Whatever may be the care wherewith the electric resistors insure heating the moistened air mixture, the temperature inside the discharge line will unavoidably get lower as said discharge line becomes longer. The temperature of that moistened air or gas mixture which leaves said chamber, will never correspond to the temperature of a mixture which is fed to the patient. In spite of safety thermostats being present, said temperature will vary within limits which are not always allowable.

The discharge line or air pipe from the chamber to the patient may not always be designed as a sterile unit. For instance, French Patent 2 250 542 discloses a device which is only interchangeable when replacing the conveying tube and the resistance-heater wire, as well as the thermometer and the moisture sensor. Such a device may thus hardly be thought of as "disposable" and does not fulfill elementary sanitary criteria.

The invention has thus for object to provide a sanitary device working under absolutely sterile conditions, wherewith air or medical gases may be fed in moistened and/or heated state to a patient, and the heating and heat-regulating components of which do not have to be renewed every time that the air- or gas-conditioning components have to be replaced for sanitary reasons.

Another very important object of the invention should be considered in the design of that device which insures an accurate temperature regulating of the moistened air mixture being delivered.

To obtain this according to the invention, the device according to the invention is characterized in that said tube has an enlarged portion forming a chamber between first and second mouthpieces, the first mouthpiece acting as an air outlet from the tube, and further leading to the patient, and the second mouthpiece acting as an air inlet to the tube, said cylinder-shaped element being fitted with said self-regulating resistors and surrounding said tube when it is connected to the tube.

In a first possible embodiment, said material is present in the shape of fibers.

In another possible embodiment, said material is present in the shape of beads.

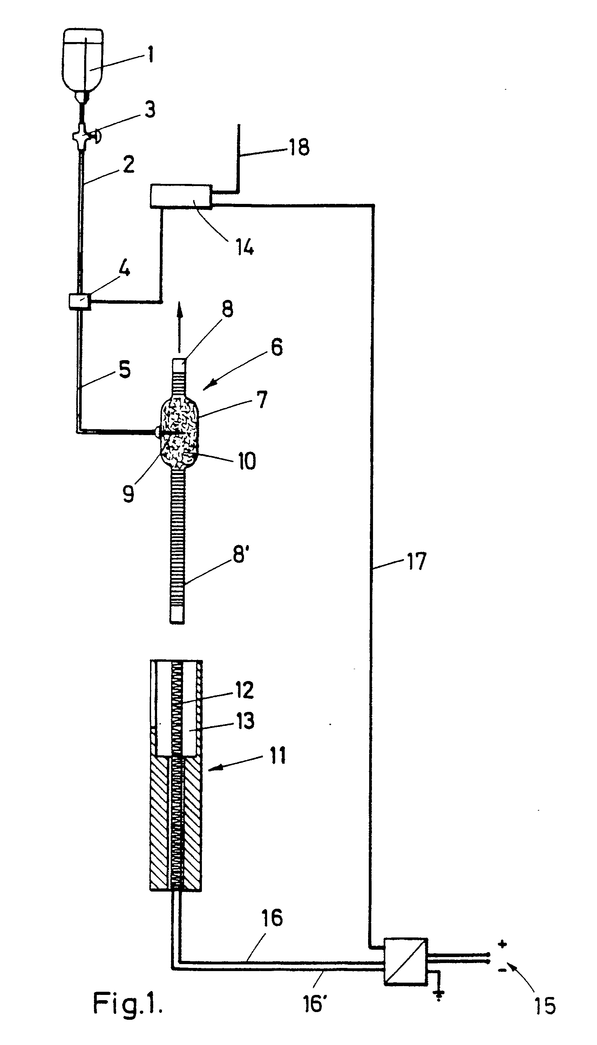

Other details and advantages of the invention will stand out from the following description, given by way of non limitative example and with reference to the accompanying drawing.

The attached figure is a diagrammatic showing of the device according to the invention, in a preferred embodiment thereof.

The device according to the invention comprises a container 1 for sterile water. Such a container may for example be a so-called "Baxter" type. An electro-magnetic throttling valve 4 is mounted on the line 2 which extends from said container 1, beyond the usual regulating cock 3. Past said throttling valve, the line 5 runs to a tube 6 with an enlarged portion which forms a chamber 7 which is provided between two mouthpieces 8 and 8'. When as it is preferred, tube 6 is made of mouthpieces, the end of line 5 may be provided with a hollow needle 9 which can be pushed through the wall of said tube, that is chamber 7 thereof.

The tube 6 should be considered as being connected in the supply to the respirator or aneasthetic apparatus, not shown, of a patient.

To let the required air volume through tube 6 and simultaneously insure optimum moistening thereof, the chamber 7 in tube 6 is filled with a material which may be provided in the shape of fibers or beads. Such material is shown with numeral 10. It is essential for the invention that said material adsorbs water on the surface or simply absorbs water, while still letting ai through unhindered.

The air inlet to the tube 6 is assured at the base of mouthpiece 8'. The upper end of moutpiece 8 can be connected with any kind of piping system leading to the patient. The arrow indicates the flow direction of the air to the patient.

The tube 6 which forms a disposable unit, is intended to be slipped into the cylinder-shaped element 11. Said cylinder-shaped element 11 comprises a self-regulating resistor 12 which runs along the passageway 13 connecting thereto of the cylinder-shaped component. Said resistor thus lies in close contact with the wall of mouthpiece 8'.

The resistor for heating the water and the air flowing through, may be provided about said cylinder-shaped element, but it might also be arranged sidewise relative to said element.

With the same principle as base, a plurality of elements fitted with self-regulating resistors may be connected together, or may be provided at the end of the "air pipe" which leads to a patient.

This allows to retain constant the temperature of the circulating moistened air and to prevent condensation. The self-regulating heating resistors naturally insure the circulating air being retained constant. Each cylinder-shaped element which is being used thereby, thus comprises a continuous passage for a tube 6 and means whereby two such elements can be coupled to one another and the heating resistors thereof be connected together.

As those materials both the cylinder-shaped element 11 and the tube 6 are made of, are flexible at room temperature, it is possible according to the requirements, to fold the cylinder-shaped element 11 with the tube provided therein. This thus allows to retain the temperature of the air flowing in the "air pipe" at the desired level, while adapting the "air pipe" profile to the requirements.

The invention further relates to the provision of a programmer 14 which controls the throttling valve 4 and is connected to the respirator or anaesthetic apparatus not shown in the figure.

In the diagrammatic showing according to the figure, there is further noticed a current source 15, lines 16 and 16' which lead to resistor 12, while reference numeral 17 shows the line to programmer 14 and numeral 18 the line to and from the device remote control.

It appears very clearly from the above description of the device according to the invention, that the device design offers the following remarkable advantages:

1) a far-driven hygiene because the required water is collected into disposable sterile containers and is supplied through similar lines;

2) the same is valid for the containers wherein that material is enclosed which adsorbs the water in the shape of microscopic droplets on the surface;

3) due to the module-like design of the tube 6, a completely constant heated air-pipe circuit can be built-up;

4) due the provision of a programmer and a throttling valve controlled thereby, the possibility is given of obtaining a programmed operation of the device by the respirator or anaesthetic apparatus connected thereto. Such operation may also be synchronized with the respirator or anaesthetic apparatus of the patient.

## Claims

1. Device for supplying air or medical gases in a conditioned, particularly a moistened and/or heated state to a patient, comprising a tube (6), wherethrough the moistened air flows, and a cylinder-shaped element (11) to which the tube (6) is connectable, self-regulating heating resistors (12) being provided which heat the air flowing through said tube (6), said tube (6) being at least partly filled with a material (10) which adsorbs or absorbs the water supplied by a water-supply line (2) from a container (1), and has a good air-permeability, characterized in that said tube (6) has an enlarged portion forming a chamber (7) between first and second mouthpieces (8, 8'), the first mouthpiece (8) acting as an air outlet from the tube (6), and further leading to the patient, and the second mouthpiece (8') acting as an air inlet to the tube (6), said cylinder-shaped element (11) being fitted with said self-regulating resistors (12) and surrounding said tube (6) when it is connected to the tube.

2. Device as defined in claim 1, characterized in that said material (10) is present in the shape of fibers.

3. Device as defined in claim 1, characterized in that said material (10) is present in the shape of beads.

4. Device as defined in any one of claims 1 to 3, characterized in that said tube (6) is made of a material wherethrough a hollow needle (9) can be pushed, which needle connects to a line for supplying water from said container (1).

5. Device as defined in any one of claims 1 to 4, characterized in that said water-supply line (2) is provided with an electro-magnetic throttling valve (4) which can be controlled by means of a programmer (14).

6. Device as defined in any one of claims 1 to 5, characterized in that said cylinder-shaped element (11) and the tube (6) mounted therein, are made of a material which is flexible at room temperature.

## Revendications

1. Dispositif pour fournir à un patient de l'air ou des gaz médicaux dans un état conditionné, en particulier humidifié et/ou chauffé, comprenant un tube (6) au travers duquel s'écoule l'air humidifié et une partie en forme de cylindre (11) à laquelle peut être raccordé le tube (6), des résistances de chauffage autorégulatrices (12) qui chauffent l'air s'écoulant au travers du tube (6) étant prévues, le tube (6) étant au moins partiellement rempli d'une matière (10) qui adsorbe ou absorbe l'eau fournie à partir d'un réservoir (1) par une conduite d'alimentation en eau (2) et qui présente une bonne perméabilité à l'air, caractérisé en ce que le tube (6) présente une portion élargie formant une chambre (7) située entre des première et seconde embouchures (8, 8'), la première embouchure (8) agissant en tant que sortie d'air à partir du tube (6) et de plus aboutissant au patient et la seconde embouchure (8') agissant en tant qu'entrée d'air pour le tube (6), la partie en forme de cylindre (11) étant équipée des résistances autorégulatrices (12) et entourant ledit tube (6) lorsqu'elle est raccordée au tube.

2. Dispositif suivant la revendication 1, caractérisé en ce que ladite matière (10) est présente sous la forme de fibres.

3. Dispositif suivant la revendication 1, caractérisé en ce que ladite matière (10) est présente sous la forme de perles.

4. Dispositif suivant l'une quelconque des revendications 1 à 3, caractérisé en ce que le tube (6) est réalisé en une matière qui peut être transpercée par une aiguille creuse (9), cette aiguille se raccordant à une conduite pour l'alimentation en eau en provenance du réservoir (1).

5. Dispositif suivant l'une quelconque des revendications 1 à 4, caractérisé en ce que la conduite d'alimentation en eau (2) est équipée d'une vanne électromagnétique d'étranglement (4) qui peut être commandée au moyen d'un programmateur (14).

6. Dispositif suivant l'une quelconque des revendications 1 à 5, caractérisé en ce que la partie en forme de cylindre (11) et le tube (1) monté dans cette dernière sont réalisés en une matière qui est flexible à la température ambiante.

**Patentansprüche**

1. Vorrichtung zur Versorgung von Patienten mit Luft oder medizinischen Gasen insbesondere in angefeuchtetem oder erhitztem Zustand, enthaltend ein Rohr (6), durch welches die angefeuchtete Luft fließt und ein eine Zylinderform aufweisendes Element (11) an das das Rohr (6) anschließbar ist mit selbstregelnden Heizwiderständen (12) die die durch das Rohr (6) fließende Luft erwärmen, wobei das Rohr (6) mindestens teilweise mit einem Material (10) gefüllt ist, das das Wasser adsorbiert oder absorbiert, welches durch eine Wasserzufuhrleitung von einem Behälter (1) zugeführt wird und eine gute Luftpermeabilität aufweist dadurch gekennzeichnet, daß das Rohr (6) einen aufgeweiteten Abschnitt aufweist, der eine Kammer (7) zwischen ersten und zweiten Mundstücken (8, 8') bildet, wobei das erste Mundstück (8) als Luftauslaß aus dem Rohr (6) wirkt und weiterführt zum Patienten und das zweite Mundstück (8') als Lufteinlaß in das Rohr (6) wirkt und wobei das zylinderförmige Element (11) mit den selbstregelnden Widerständen versehen ist und das Rohr (6) umfaßt, wenn es an dieses angeschlossen ist.

2. Vorrichtung nach Anspruch 1 dadurch gekennzeichnet, daß das Material (10) in Form von Fasern vorliegt.

3. Vorrichtung nach Anspruch 1 dadurch gekennzeichnet, daß das Material (10) in Form eines Bettes vorliegt.

4. Vorrichtung nach einem der Ansrüche 1 - 3 dadurch gekennzeichnet, daß das Rohr (6) aus einem Material besteht, durch das eine Hohlnadel einführbar ist, wobei die Nadel an eine Wasserzufuhrleitung aus dem Behälter (1) angeschlossen ist.

5. Vorrichtung nach einem der Ansprüche 1 - 4 dadurch gekennzeichnet, daß die Wasserzufuhrleitung (2) mit einem elektromagnetischen Drosselventil (4) versehen ist, das durch einen Rechner (14) steuerbar ist.

6. Vorrichtung nach einem der Ansprüche 1 - 5 dadurch gekennzeichnet daß das zylinderförmige Element (11) und das Rohr (6) aus einem Material bestehen, das bei Raumtemperatur flexibel ist.

Fig.1.